# EUROPEAN PATENT APPLICATION

(11) **EP 2 455 364 A1**
(43) Date of publication of application: **23.05.2012**
(21) Application number: 10191863.9
(22) Date of filing: 19.11.2010
(51) Int. Cl.: C07C 249/02, C07C 319/24, C07D 213/127, C07D 213/53, C07D 307/52, C07D 333/22

(54) **Process for Preparing Azomethines from alpha-Oxocarboxylates, Amines and Aryl Bromides**

(71) Applicant: Saltigo GmbH, 40764 Langenfeld (DE)
(72) Inventor: Cotté, Alain, D-51377 Leverkusen (DE); Gotta, Matthias, D-51399 Burscheid (DE); Goossen, Lukas, D-67663 Kaiserslautern (DE); Rudolphi, Felix, D-67663 Kaiserslautern (DE); Song, Bingrui, D-67655 Kaiserslautern (DE)
(74) Representative: Pettrich, Klaus-Günter

(57) **Abstract**

A process for preparing azomethines of the general formula (V) 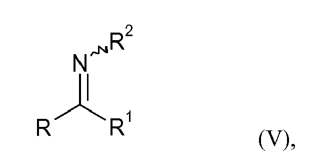 where
R is an optionally substituted carbocyclic aromatic radical having 6 to 24 carbon atoms or an optionally substituted alkyl radical or an optionally substituted heteroaromatic radical having 5 to 24 carbon atoms, and
R¹ is an optionally substituted carbocyclic aromatic radical having 6 to 24 carbon atoms or an optionally substituted heteroaromatic radical having 5 to 24 carbon atoms,
R² is hydrogen or an optionally substituted carbocyclic aromatic radical having 6 to 24 carbon atoms or an optionally substituted alkyl radical or an optionally substituted cycloalkyl radical or an optionally substituted heteroaromatic radical having 5 to 24 carbon atoms

by reacting alpha-oxo carboxylates of the general formula (I) 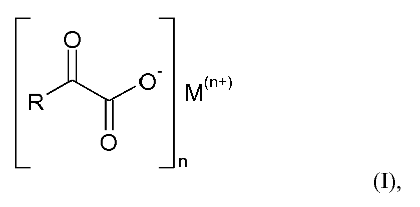 where
n is a number in the range from 1 to 6,
M⁽ⁿ⁺⁾ is a cation,

with aryl bromides of the general formula (IV)

R¹-Br (IV)

and amines of the general formula (II)

R²-NH₂ (II)

via the alpha-iminocarboxylate intermediate of the general formula (III), 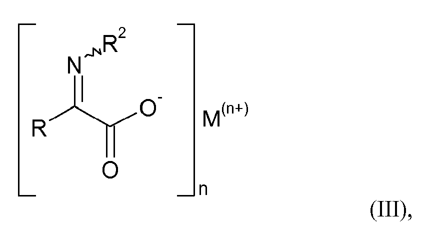 in the presence of two transition metals or compounds thereof as catalyst, is described.

## Description

The present invention relates to a novel process for preparing azomethines (Schiff bases) from alpha-oxocarboxylates, amines and aryl bromides by Cu/Pd-catalysed decarboxylating cross-coupling.

Imines are valuable intermediates in the synthesis of amines and nitrogen-containing heterocycles. Moreover, imino groups are widely utilized as directing groups in aromatic C-H activation reactions and are key functionalities in ligands for olefin polymerization catalysis. A direct, yet modular access to this compound class based on easily available reagents, in which all three substituents can be independently varied, is thus of high interest.

Traditional syntheses of imines include the condensation of the respective carbonyl compound with a primary amine (R. W. Layer, Chem. Rev. 1963, 63, 489-510), the addition of organometallic reagents to nitriles (G. E. P. Smith Jr., F. W. Bergstrom, J. Am. Chem. Soc. 1934, 56, 2095-2098; R. L. Garner, L. Hellerman, J. Am. Chem. Soc. 1946, 68, 823-825), and the arylation of nitriles under Friedel-Crafts conditions (K. C. Gulati, S. R. Seth, K. Venkataraman, Org. Synth. 1935, 15, 70-71). However, none of these syntheses is fully modular, as the products result from combinations of only two reagents. Moreover, they are often incompatible with sensitive functional groups due to the harsh reaction conditions or the use of organometallic reagents. Examples of modern two-component syntheses are the catalytic hydroamination of alkynes (M. Beller, J. Seayad, A. Tillack, H. Jiao, Angew. Chem. 2004, 116, 3448-3479; Angew. Chem. Int. Ed. 2004, 43, 3368-3398), the ruthenium-catalyzed oxidative coupling of aldimines and boronates (Y. J. Park, E. A. Jo, C. H. Jun, Chem. Commun. 2005, 1185-1187), the palladium-catalyzed addition of arenes or boronic acids to nitriles (C. Zhou, R. C. Larock, J. Org. Chem. 2006, 71, 3551-3558; Y. C. Wong, K. Parthasarathy, C. H. Cheng, Org. Lett. 2010, 12, 1736-1739) and the dehydrogenative coupling of alcohols and amines (J. Zhang, D. Milstein, Angew. Chem. 2010, 122, 1510-1513; Angew. Chem. Int. Ed. 2010, 49, 1468-1471). One of the few three-component imine syntheses consists of the catalytic cross-coupling of aryl halides with organometallic reagents in the presence of toxic and malodorous isonitriles (T. Ishiyama, T. Oh-e, N. Miyaura, A. Suzuki, Tetrahedron Lett. 1992, 33, 4465-4468).

It has previously been demonstrated in the synthesis of biaryls from benzoic acid salts and aryl halides that decarboxylating cross-couplings may represent valuable alternatives to corresponding reactions of organometallic compounds (WO 2006/136135; L. J. Gooßen, N. Rodriguez, P. P. Lange, C. Linder, Angew. Chem. 2010, 122, 1129-1132; Angew. Chem. Int. Ed. 2010, 49, 1111-1114). Also Decarboxylative couplings have recently emerged as a powerful synthetic strategy to access to aryl ketones (L. J. Gooßen, F. Rudolphi, C. Oppel, N. Rodriguez, Angew. Chem. 2008, 120, 3085-3088; Angew. Chem. Int. Ed. 2008, 47, 3043-3045; P. Fang, M. Li, H. Ge, J. Am. Chem. Soc. 2010, 132, 11898-11899) and esters (R. Shang, Y. Fu, J. B. Li, S. L. Zhang, Q. X. Guo, L. Liu, J. Am. Chem. Soc. 2009, 131, 5738-5739). The inherent advantage of these redox-neutral couplings is that simple carboxylic acid salts replace preformed organometallic reagents as the sources of carbon nucleophiles.

It was an object of the present invention to find a process for preparing azomethines (Schiff bases), which start from readily available compounds and employ easily handled and low-cost catalysts.

A new process for the preparation of the azomethine moiety was found involving the decarboxylative coupling of aryl halides and alpha-iminocarboxylates, generated in situ from alpha-oxocarboxylate salts and primary amines. Implementing this new procedure allows to reduce the temperature of the cross coupling reaction as the activation barrier for the decarboxylation step of alpha-iminocarboxylate salts is lower than for the corresponding alpha-oxocarboxylates. It is possible to combine the condensation of the amine with the reactive carbonyl group of an alpha-oxocarboxylate to give the alpha-iminocarboxylate and its decarboxylative cross-coupling reaction with aryl halides into a one-pot process. In combination with an optional hydrolytic cleavage of the azomethines, the same process would also give access to aryl ketones or additional to secondary or tertiary amines by reduction of the Schiff bases.

The invention therefore relates to a process for preparing azomethines of the general formula (V) 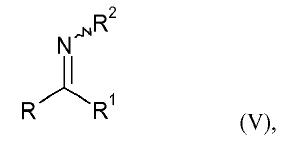 where
R is an optionally substituted carbocyclic aromatic radical having 6 to 24 carbon atoms or an optionally substituted alkyl radical or an optionally substituted heteroaromatic radical having 5 to 24 carbon atoms, and
R¹ is an optionally substituted carbocyclic aromatic radical having 6 to 24 carbon atoms or an optionally substituted heteroaromatic radical having 5 to 24 carbon atoms,
R² is hydrogen or an optionally substituted carbocyclic aromatic radical having 6 to 24 carbon atoms or an optionally substituted alkyl radical or an optionally substituted cycloalkyl radical or an optionally substituted heteroaromatic radical having 5 to 24 carbon atoms
by reacting alpha-oxocarboxylates of the general formula (I) 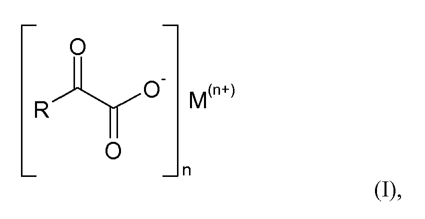 where
n is a number in the range from 1 to 6,
M⁽ⁿ⁺⁾ is a cation, and
R has the meaning indicated for formula (V),
with aryl bromides of the general formula (IV)

R¹-Br (IV)

where
R¹ has the meaning indicated for formula (V),
and amines of the general formula (II)

R²-NH₂ (II)

where
R² has the meaning indicated for formula (V)
via the alpha-iminocarboxylate intermediate of the general formula (III), 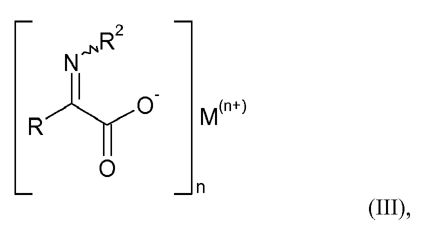 where
n is a number in the range from 1 to 6,
M⁽ⁿ⁺⁾ is a cation, and
R has the meaning indicated for formula (V),
R² has the meaning indicated for formula (II),
in the presence of two transition metals or compounds thereof as catalyst.

In the first step, the alpha-oxocarboxylate (I) would react with the primary amine (II) to the alpha-iminocarboxylate (III). Decarboxylation of the alpha-iminocarboxylate in the presence of two transition metals or compounds thereof, for example in the presence of a Cu catalyst in combination with a Pd-mediated cross-coupling with the aryl bromide of the formula (IV) results in the azomethine of the formula (V):

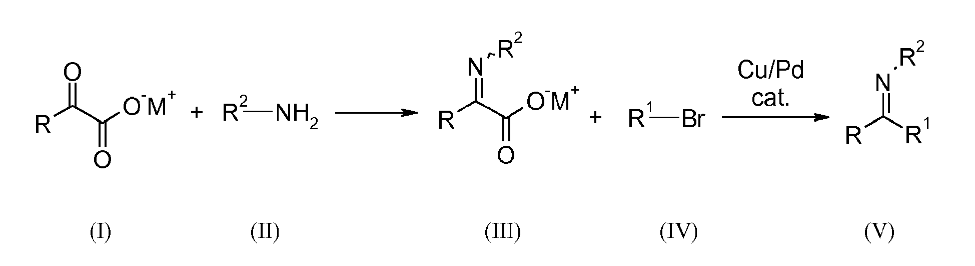

The advantage of this decarboxylating cross-coupling to prepare azomethines is that, in contrast to traditional cross-coupling reactions, no organometallic reagents are necessary. Instead, easily handled, readily obtainable stable salts of alpha-oxo carboxylic acids, some of which are available industrially as intermediates in the preparation of amino acids, are used as source of acylnucleophiles.

The catalyst system in the process according to the invention comprises two metal components. The first component comprises a metal which can assume two oxidation states differing by one unit, particularly preferably from the series Ag (0,I), Cu (0, I, II), Mn (II, III), Fe (II, III), Co (II, III), Ni (II, III), Mo (IV, V), Ru (II, III) (suitable combinations of oxidation states by way of example in parentheses). The metal can optionally be employed in elemental form, as complex or as salt. Copper(I) compounds are particularly preferably employed, and copper(I) bromide is very particularly preferably employed as one of the transition metal components.

The second component of the catalyst system comprises a transition metal which can assume two oxidation states differing by two units, preferably from the series Pd (0, II), Ni (0, II), Fe (-II, 0,II), Au (I, III), Rh (I, III), Pt (0, II, IV), Ru (0, II), Ir (I, III) (suitable combinations of oxidation states by way of example in parentheses). The metal can optionally be employed in elemental form, as complex or as salt. Platinum metals are particularly preferably employed, palladium compounds are very particularly preferably employed, and palladium(II) bis(1,1,1,5,5,5-hexafluoroacetylacetonate) (Pd(F₆-acac)₂) is even more preferably employed as the second transition metal component.

Both metals can independently of one another optionally be stabilized by further ligands, preferably from the series amines, phosphines, N-heterocyclic carbenes, nitriles or olefins. Cyclic amines are particularly preferably used as ligands, and chelating cyclic amines from the series phenanthroline, bipyridine and terpyridine or their substituted derivatives are very particularly preferably used.

The addition of phosphine ligands has an advantageous influence on the reaction, and diphosphine ligands are preferably, and 1,1'-bis(diphenylphosphino)ferrocene (dppf) is particularly preferably employed.

It is optionally possible for the actual catalysts to be generated in the reaction mixture from suitable metal precursors by adding the components (ligands).

The catalyst system particularly preferably employed for the process according to the invention is a combination of copper(I) bromide with 1,10-phenanthroline as ligand and Pd(F₆acac)₂ with 1,1'-bis(diphenylphosphino)ferrocene (dppf) as ligand.

In the process according to the invention, the two catalysts are employed independently of one another in amounts of from 0.001 mol% to 50 mol% based on the carbon electrophile (R¹-Br), and amounts of from 0.01 mol% to 15 mol% are preferably employed.

The process according to the invention is carried out at temperatures from 20°C to 180°C, preferably at 60°C to 140°C and particularly preferably at 80°C to 120°C.

The process according to the invention is normally carried out in the presence of a solvent. Solvents which can be employed for example are one or mixtures of the starting materials, aromatic hydrocarbons (for example benzene, toluene, xylenes, ethylbenzene, mesitylene), ethers (for example 1,4-dioxane, tetrahydrofuran, methyltetrahydrofuran, dibutyl ether, methyl t-butyl ether, diisopropyl ether, diethylene glycol dimethyl ether), amides (for example dimethylformamide, diethylformamide, N-methylpyrrolidone, dimethylacetamide), aromatic amines (quinoline, pyridine), dimethyl sulphoxide, sulpholane, acetonitrile, isobutyronitrile, propionitrile, propylene carbonate and chlorinated aliphatic and aromatic hydrocarbons. Amides (for example dimethylformamide, N-methylpyrrolidone) and aromatic amines (for example quinoline, pyridine) or amide/amine mixtures are preferably employed.

The process according to the invention can preferably be carried out in such a way that traces of water can be removed by continuous azeotropic distillation during the reaction or by the addition of a desiccant. It is also possible to operate under pressure to achieve the necessary reaction temperature.

R, R¹ and R² are in the context of the invention preferably carbocyclic aromatic radicals having 6 to 24 framework carbon atoms or heteroaromatic radicals having 5 to 24 framework carbon atoms, in which zero, one, two or three carbon atoms in the framework of each ring, but at least one framework carbon atom in the complete molecule, may be replaced by heteroatoms selected from the group of nitrogen, sulphur or oxygen. The carbocyclic aromatic radicals or heteroaromatic radicals may furthermore be substituted by up to five identical or different substituents in each ring, selected from the group of hydroxy, halogen, nitro, amino, mercapto, cyano, free or protected formyl, C₁-C₁₂-alkyl, C₁-C₁₂-haloalkyl, C₁-C₁₂-alkylthio; C₅-C₁₄-aryl, C₆-C₁₅-arylalkyl, C₁-C₁₂-alkoxy and C₁-C₁₂-alkoxycarbonyl. R¹ is for example phenyl, tolyl, thienyl or naphthyl which is optionally substituted once, twice or three times by radicals which are each selected independently of one another from the group of C₁-C₆-alkyl, C₁-C₆-haloalkyl, C₁-C₆-alkylthio, C₅-C₁₄-aryl, C₁-C₆-alkoxy, C₁-C₆-alkoxycarbonyl, halogen, hydroxy, nitro, amino, mercapto and cyano. R¹ is preferably a tolyl, cyanophenyl or methoxyphenyl radical.

R is additionally in the context of the invention preferably alkyl, in each case independently is an optionally substituted straight-chain, cyclic, branched or unbranched alkyl radical. Examples of suitable substituents for the alkyl radical are C₁-C₆-alkyl, C₁-C₆-haloalkyl, C₅-C₁₄-aryl, C₆-C₁₅-arylalkyl, C₁-C₆-alkoxy, C₁-C₆-aryloxy, C₁-C₆-alkoxycarbonyl, C₁-C₆-acyloxy or halogen. Alkyl is particularly preferably for example n-propyl, isopropyl, n-butyl, tert-butyl, isobutyl, n-pentyl or cyclohexyl. R is preferably a phenyl, cyanophenyl or tert-butyl radical.

R² is additionally in the context of the invention preferably alkyl, in each case independently is an optionally substituted straight-chain, cyclic, branched or unbranched alkyl radical. Examples of suitable substituents for the alkyl radical are C₁-C₆-alkyl, C₁-C₆-haloalkyl, C₃-C₆-cycloalkyl, C₅-C₁₄-aryl, C₆-C₁₅-arylalkyl, C₁-C₆-alkoxy, C₁-C₆-aryloxy, C₁-C₆-alkoxycarbonyl, C₁-C₆-acyloxy or halogen. Cycloalkyl is particularly preferably for example cyclopentyl or cyclohexyl, R² is preferably a cyclohexyl radical.

The counter-ion M⁽ⁿ⁺⁾ from formula (I) is in this connection a metal cation which leads to equalization of charges, preferably from the series of metals Li, Na, K, Mg, Ca, B, Al, Ag, Cu, Mn, Fe, Co, Ni, Mo, Ru, particularly preferably from the series of sodium or potassium (n = 1).

The alpha-oxo carboxylic acid salts of the formula (I) are optionally added in preformed form or are generated in situ from the alpha-oxo carboxylic acids and suitable bases.

The new process of the invention can also be utilized for the synthesis of aryl ketones, as the azomethine products are easily hydrolyzed, e.g. by adding HCl_{aq} after the reaction is complete. The synthesis of aryl ketones from α-oxocarboxylates by their in situ conversion to imines, cross-coupling and subsequent hydrolysis of the azomethine intermediates is advantageous over the direct coupling of the α-oxocarboxylates because of the dramatically reduced reaction temperatures (100°C rather than 170°C). Therefore a further object of the invention is a process for the synthesis of aryl ketones by hydrolyzing an azomethine prepared as described above. The hydrolysis is carried out by adding a mineral acid such as HCl_{aq} or H₂SO₄ or water to the azomethine.

### Examples:

### Examples (Va to Vz)

The following catalyst systems were used for the examples:
15 mol% copper(I) bromide, 15 mol% 1,10-phenanthroline, 2 mol% dppf and 1 mol% Pd(F₆-acac)₂, at 100°C for 16 hours in NMP which successfully converts a large number of aryl bromides with various alpha-oxo carboxylic acids and a variety of primary amines into azomethines

Course of the reaction:

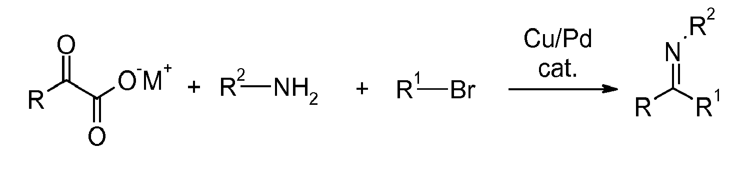

| Product | Yield /% | Product | Yield /% |
|---|---|---|---|
| 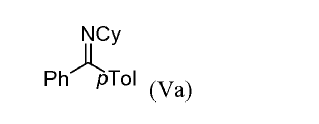 | 91 | 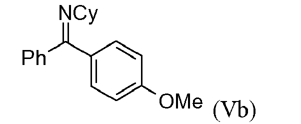 | 90 |
| 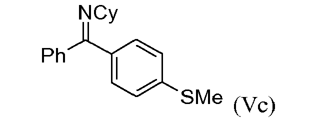 | 92 | 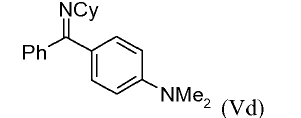 | 85 |
| 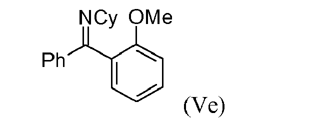 | 86 | 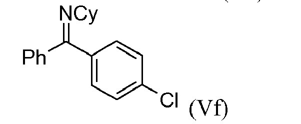 | 94 |
| 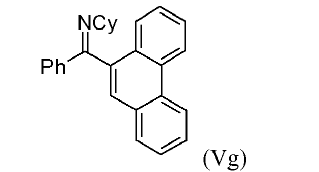 | 66 | 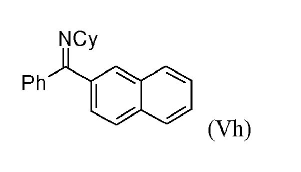 | 93 |
| 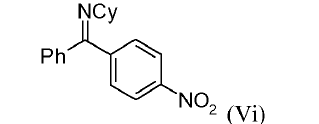 | 57 | 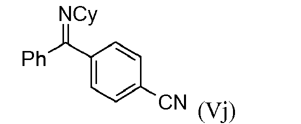 | 95 |
| 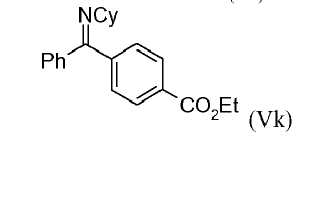 | 83 | 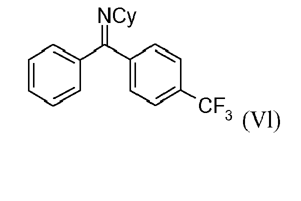 | 65 |
| 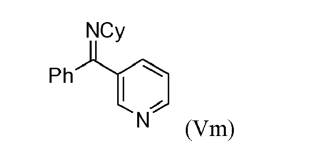 | 91 | 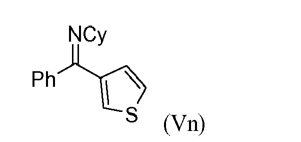 | 85 |
| 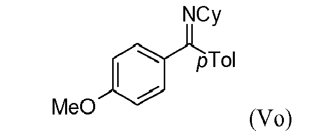 | 34 | 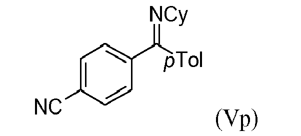 | 94 |
| 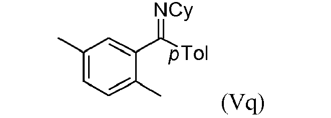 | 66 | 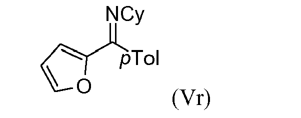 | 71 |
| 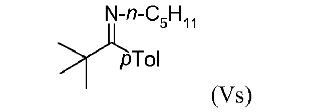 | 45 | 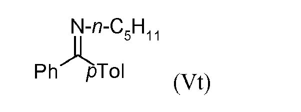 | 87 |
| 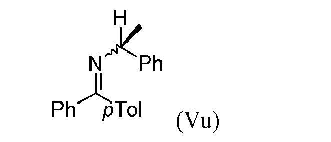 | 64 | 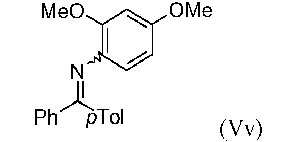 | 94 |
| 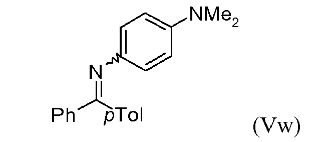 | 60 | 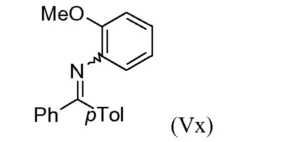 | 73 |
| 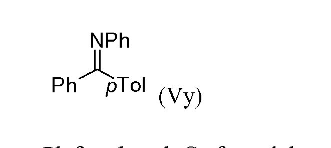 | 53 | 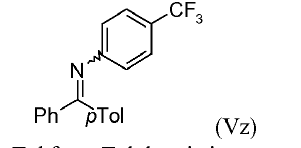 | 16 |

| | | | |
|---|---|---|---|
| Ph tor phenyl, Cy tor cylohexyl and *p*-Tol for *p*-Tolyl moieties. | | | |

The substituent from the alpha-oxo carboxylic acid is drawn to the left of the carbonyl group in the Table.

### Example (Va)

**Synthesis of *N*-(phenyl-(4-tolyl)-methylene)-cyclohexylamine (Va).** A mixture of potassium phenyloxoacetate (Ia, 4.52 g, 24.0 mmol), palladium(II) 1,1,1,3,3,3-hexafluoroacetylacetonate (104 mg, 0.20 mmol), copper(I) bromide (430 mg, 3.00 mmol) under nitrogen was treated with a solution of cyclohexylamine (IIa, 2.38 g, 2.75 ml, 24.0 mmol), 4-bromotoluene (IVa, 3.42 g, 2.46 mL, 20.0 mmol), 1,1'-diphenylphosphinoferrocene (114 mg, 0.20 mmol), and 1,10-phenanthroline (541 mg, 3.00 mmol) in 40 mL NMP and 10 mL cyclohexane, excluding air and moisture. The reaction mixture was refluxed at 100 °C for 16 h. It was then washed twice with saturated sodium bicarbonate solution, water, and once with brine, and the aqueous phases were extracted twice with ethyl acetate. The combined organic phases were dried over magnesium sulfate and filtered. After removal of the solvent followed by destillation (130 °C / 4x10⁻³ mbar), the product was obtained as yellow oil (4.58 g, 83% yield).

It can be inferred from that the alpha-oxo carboxylic acids react both with electron-rich and electron-poor aryl and heteroaryl bromides to give the azomethines in good yields, with many functional groups being tolerated, inter alia esters, nitros and nitriles.

On the other hand, 4-bromotoluene could be coupled in good yields with various alkyl-, aryl- and heteroaryl-substituted alpha-oxo carboxylic acids using different primary amines, and therefore the broadly applicable novel synthesis method for preparing aryl azomethines was demonstrated. It was even possible to allows the coupling of aryl chlorides using 2-(dicyclohexylphosphino)-2',4',6'-triisopropylbiphenyl instead of dppf as exemplified by the synthesis of Va in 61% yield from 4-chlorotoluene.

## Claims

1. Process for preparing azomethines of the general formula (V) 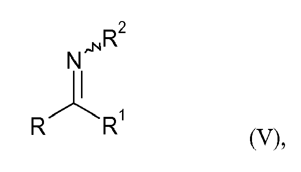 where
R is an optionally substituted carbocyclic aromatic radical having 6 to 24 carbon
atoms or an optionally substituted alkyl radical or an optionally substituted heteroaromatic radical having 5 to 24 carbon atoms, and
R¹ is an optionally substituted carbocyclic aromatic radical having 6 to 24 carbon
atoms or an optionally substituted heteroaromatic radical having 5 to 24 carbon atoms,
R² is hydrogen or an optionally substituted carbocyclic aromatic radical having 6 to 24
carbon atoms or an optionally substituted alkyl radical or an optionally substituted cycloalkyl radical or an optionally substituted heteroaromatic radical having 5 to 24 carbon atoms
by reacting alpha-oxocarboxylates of the general formula (I) 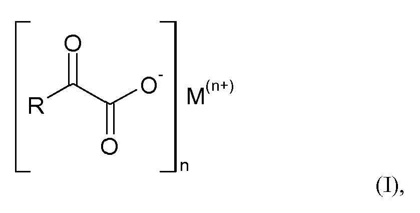 where n is a number in the range from 1 to 6,
M⁽ⁿ⁺⁾ is a cation, and
R has the meaning indicated for formula (V),
with aryl bromides of the general formula (IV)
R¹-Br (IV)
where
R¹ has the meaning indicated for formula (V),
and amines of the general formula (II)
R²-NH₂ (II)
where
R² has the meaning indicated for formula (V)
via the alpha-iminocarboxylate intermediate of the general formula (III), 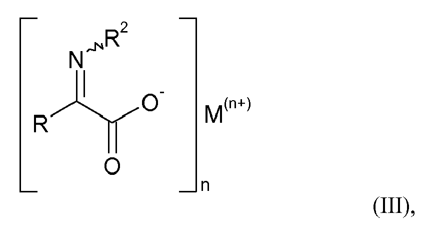 where
n is a number in the range from 1 to 6,
M⁽ⁿ⁺⁾ is a cation, and
R has the meaning indicated for formula (V),
R² has the meaning indicated for formula (V),
in the presence of two transition metals or compounds thereof as catalyst.

2. Process according to Claim 1, where the catalyst comprises two different transition metals and/or transition metal compounds, **characterized in that** one transition metal or one transition metal compound is selected from those able to assume oxidation states differing from one another by one unit, and the other transition metal or the other transition metal compound is selected from those able to assume oxidation states differing from one another by two units.

3. Process according to either of Claims 1 or 2, where the one transition metal or the transition metal compound is selected from the series of metals Ag, Cu, Mn, Fe, Co, Ni, Mo, Ru and compounds thereof and the other transition metal or the other transition metal compound is selected from the series of metals Pd, Ni, Fe, Au, Rh, Pt, Ru, Ir and compounds thereof.

4. Process according to any of Claims 1-3, where at least one of the transition metals is stabilized by ligands from the series of amines, N-heterocyclic carbenes, nitriles, olefins or phosphines.

5. Process according to Claim 4, **characterized in that** the ligand is a chelating amine from the series phenanthroline, bipyridine, terpyridine and/or a trialkylphosphine.

6. Process according to Claim 1, **characterized in that** a combination of copper(I) bromide with 1,10-phenanthroline as ligand and bis(1,1,1,5,5,5-hexafluoroacetylacetonato)-palladium with 1,1'-bis(diphenylphosphino)ferrocene (dppf) as ligand is employed as catalyst.

7. Process according to any of Claims 1 to 5, where the amounts of the two catalysts employed independently of one another are from 0.001 mol% to 50 mol% based on the compound of the formula (IV).

8. Process according to any of Claims 1 to 7, **characterized in that** the process is carried out at temperatures from 60°C to 140°C.

9. Process for the synthesis of aryl ketones **characterized in that** an azomethine prepared according to any of Claims 1 to 8 is hydrolyzed.
